(19) Europäisches Patentamt

European Patent Office

Office européen des brevets

(11) **EP 1 045 245 A2**

(12) **EUROPÄISCHE PATENTANMELDUNG**

(43) Veröffentlichungstag:
**18.10.2000 Patentblatt 2000/42**

(51) Int. Cl.[7]: **G01N 33/18**

(21) Anmeldenummer: **00107735.3**

(22) Anmeldetag: **11.04.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **14.04.1999 DE 19916792**

(71) Anmelder: **Dr. THIEDIG + CO.**
**D-13357 Berlin (DE)**

(72) Erfinder:
• **Plug, Hans D., Dr.**
  **60388 Frankfurt (DE)**
• **Maughan, Eric.**
  **47198 Duisburg (DE)**

(74) Vertreter:
**Weisse, Jürgen, Dipl.-Phys. et al**
**Patentanwälte,**
**Dipl.-Phys. Jürgen Weisse,**
**Dipl.-Phys. Dr. Ing. Renate Weisse,**
**Bökenbusch 41**
**42555 Velbert (DE)**

(54) **Verfahren und Vorrichtung zur Überwachung von ammoniakalisierten Wasserdampfkreisläufen**

(57) Die Erfindung betrifft ein Verfahren und eine Vorrichtung zur analytischen Bestimmung von chemischen Parameter in ammioniakalisierten Wasser- bzw. Wasserdampf-Kreisläufen, bei welchem ein Probestrom dem Kreislauf entnommen wird. Eine erste Leitfähigkeits-Meßvorrichtung (22) befindet sich stromaufwärts von einem Kationenaustauscher (30). Eine zweite Leitfähigkeits-Meßvorrichtung (24) befindet sich stromabwärts von dem Kationenaustauscher (30). Mittels der Leitfähigkeits-Meßvorrichtungen (22,24) wird die Leitfähigkeit durch Steuerung mittels eines Mikroprozessors (58) gemessen und gewisse chemische Parameter rechnerisch ermittelt. Insbesondere wird dabei die Ammoniak-Konzentration, die Kohlensäure-Konzentration und der pH-Wert rechnerisch ermittelt.

FIG. 1

EP 1 045 245 A2

Printed by Xerox (UK) Business Services
2.16.7 (HRS)/3.6

## Beschreibung

### Technisches Gebiet

Die Erfindung betrifft ein Verfahren nach dem Oberbegriff des Anspruchs 1 sowie eine Vorrichtung nach dem Oberbegriff des Anspruchs 20.

Bei einer großen Anzahl von Wasserdampfkreisläufen in fossilbefeuerten und nuklearen Kraftwerken wird Ammoniak-Lösung zur Minderung von Korrosionsprozessen zugegeben. Dabei ist die analytische Überwachung solcher ammoniakalisierten Wasserdampfkreisläufe von großer Bedeutung. Zur Durchführung der Überwachung werden Probeströme aus der Wasserdampfleitung kontinuierlich entnommen und hinsichtlich gewissen chemischen Parametern, wie elektrische Leitfähigkeit, pH-Wert, Natriumionen-Konzentration und Temperatur analysiert.

Das erfindungsgemäße Verfahren und die erfindungsgemäße Vorrichtung können hierbei Verwendung finden.

Bei bekannten Verfahren dieser Art werden die chemischen Parameter mittels hierfür geeigneten Meßinstrumenten gemessen und ausgewertet. Dieses Messen und Auswerten auf konventionelle Weise ist sehr aufwendig.

Der Erfindung liegt die Aufgabe zugrunde, die Überwachung von ammoniakalisierten Wasserdampfkreisläufen zu vereinfachen.

Der Erfindung liegt weiterhin die Aufgabe zugrunde, die Genauigkeit der Überwachung von ammoniakalisierten Wasserdampfkreisläufen zu verbessern.

Bezüglich dem Verfahren werden diese Aufgaben erfindungsgemäß durch die kennzeichnenden Merkmale des Anspruchs 1 gelöst.

Bezüglich der Vorrichtung werden diese Aufgaben erfindungsgemäß durch die kennzeichnenden Merkmale des Anspruchs 20 gelöst.

Durch das erfindungsgemäße Verfahren und die erfindungsgemäße Vorrichtung werden also gewisse chemische Parameter im Probestrom direkt aus der Messung der Leitfähigkeit vor und hinter einem stark sauren Kationenaustauscher rechnerisch ermittelt. Dabei wird dieses rechnerische Ermitteln durch das Vorsehen eines oder mehrerer Mikroprozessoren unterstützt. Die Steuerung und Datenauswertung durch Mikroprozessoren erlaubt ein sehr schnelles Ermitteln der relevanten chemischen Parameter. Dadurch kann die Prozeßführung der Überwachung des Wasserdampfkreislaufes schneller und genauer durchgeführt werden.

Ein rechnerisch zu ermittelnder, chemischer Parameter kann beispielsweise die Konzentration von Ammoniak sein. Dieser Parameter ist natürlich bei ammoniakalisierten Wasserdampfkreisläufen sehr wichtig, da es wünschenswert ist, diese Konzentration möglichst konstant zu halten.

Es kann jedoch ebenfalls von großer Bedeutung sein, weitere chemische Parameter des Wasserdampfkreislaufes zu ermitteln. Beispielsweise spielt verfahrensbedingt die Konzentration von Kohlensäure eine große Rolle. Beim Abstellen eines Kraftwerks durch das atmosphärische Vakuumbrechen der Turbine dringt nämlich Kohlensäure in das System ein. Damit wird die Chemie des Wasserdampfkreislaufes makroskopisch im wesentlichen durch das Gleichgewichtssystem Ammoniak-Kohlensäure-Wasser bestimmt. Das Vorhandensein von Kohlensäure kann also die Messungen der relevanten chemischen Parameter im Probestrom entscheidend beeinflussen. Diese Konzentration von Kohlensäure konnte bei den bekannten Verfahren des Standes der Technik bisher nicht berücksichtigt werden. In einem besonderen Teilaspekt der Erfindung beruht sie demgemäß auf der Erkenntnis, daß es vorteilhaft ist, die Konzentration von Kohlensäure zu ermitteln und weiterhin, daß es tatsächlich möglich ist, die Konzentration von Kohlensäure in ional und gasförmig gelöster Form in dem Probestrom aus Messungen der elektrischen Leitfähigkeit des Probestroms vor und hinter einem stark sauren Kationenaustauscher rechnerisch zu ermitteln.

Weiterhin kann der pH-Wert in dem Probestrom ebenfalls aus Messungen der elektrischen Leitfähigkeit des Probestroms vor und hinter dem stark sauren Kationenaustauscher rechnerisch ermittelt werden.

Das rechnerische Ermitteln der Konzentration von Ammoniak und Kohlensäure sowie des pH-Wertes aus den Messungen der elektrischen Leitfähigkeit fußt auf der Überlegung, daß im unbeeinflußten Wasserdampfmedium, d.h. in diesem Fall stromauf von dem Kationenaustauscher, die Komponenten $NH_3$, $NH_4^+$, $CO_2$, $HCO_3^-$, $CO_3^{2-}$, $H^+$ und $OH^-$ vorliegen, deren ionale Anteile zur Leitfähigkeit beitragen. Hinter einem stark sauren Kationenaustauscher, welcher Ammoniak aus dem Medium entfernt, liegen jedoch die Komponenten $CO_2$, $HCO_3^-$, $CO_3^{2-}$, $H^+$ und $OH^-$ vor, deren ionale Anteile zur Leitfähigkeit beitragen. Da die Gesamtkonzentration an Kohlensäure vor und hinter dem Kationenaustauscher gleich ist, kann sie hieraus berechnet werden. Wegen der relativ geringen Konzentrationen können die Aktivitätskoeffizienten gleich Eins gesetzt werden. Damit können die unbekannten Gesamtkonzentrationen von Ammoniak $Q_N$ und Kohlensäure $Q_C$ sowie der pH-Wert aus folgende Beziehungen berechnet werden:

a) Leitfähigkeit hinter Kationenaustauscher:

$$Q_C = {}^{c^*}CO_2 + {}^{c^*}HCO_3^{-} \tag{1}$$

$$c^* H^+ = c^* OH^- + c^* HCO_3^- + 2\, c^* CO_3^{2-} \tag{2}$$

$$c^* CO_2 = \frac{c^* H^+ \cdot c^* HCO_3^-}{K_{C1}} \tag{3}$$

$$c^* OH^- = \frac{K_W}{c^* H^+} \tag{4}$$

$$pH < 8,3 \rightarrow c^* CO_3^{2-} \approx 0$$

$$\lambda = c^* H^+ \cdot \Lambda_H + c^* OH^- \cdot \Lambda_{OH} + c^* HCO_3^- \cdot \Lambda_{HCO3} \tag{5}$$

$$\lambda = c^* H^+ (\Lambda_H + \Lambda_{HCO3}) + \frac{K_W}{c^* H^+}(\Lambda_{OH} - \Lambda_{HCO3}) \tag{6}$$

$$u = \Lambda_H + \Lambda_{HCO3}$$

$$v = \Lambda_{OH} - \Lambda_{HCO3}$$

$$Q_C = \left\{ \frac{c^* H^+}{K_{C1}} + 1 \right\} \cdot \left\{ c^* H^+ - \frac{K_W}{c^* H^+} \right\} \tag{7}$$

Auflösen von (6) nach $c^* H^+$:

$$c^* H^+ = \frac{\lambda + \sqrt{\lambda^2 + 4uv \cdot K_W}}{2u} \text{ (nur positives Vorzeichen ist sinnvoll)} \tag{8}$$

Einsetzen von $c^* H^+$ in (7) führt zu $Q_C$.

b) Leitfähigkeit vor Kationenaustauscher:

$$Q_C = c CO_2 + c HCO_3^- + c HO_3^{2-} \tag{9}$$

$$Q_N = c NH_3 + c NH_4^+ \tag{10}$$

$$c CO_2 = \frac{c H^+ \cdot c HCO_3^-}{K_{C1}} \tag{11}$$

$$c CO_3^{2-} = \frac{c HCO_3^- \cdot K_{C2}}{c H^+} \tag{12}$$

$$c NH_3 = \frac{c NH_4^+ \cdot K_N}{c H^+} \tag{13}$$

$$c_{OH^-} = \frac{K_W}{c_{H^+}} \tag{14}$$

$$c_{H^+} + c_{NH_4^+} = c_{OH^-} + c_{HCO_3^-} + 2\,c_{CO_3^{2-}} \tag{15}$$

$$pH > 8,3 \rightarrow c_{CO_2} \approx 0 \text{ und } c_{H^+} \approx 0$$

$$\kappa = c_{OH^-} \cdot \Lambda_{OH} + c_{HCO_3^-} \cdot \Lambda_{HCO3} + c_{CO_3^{2-}} \cdot \Lambda_{CO3} + c_{NH_4^+} \cdot \Lambda_{NH4} \tag{16}$$

Einsetzen (12) in (9) führt zu

$$c_{HCO_3^-} = \frac{c_{H^+} \cdot Q_C}{c_{H^+} + K_{C2}} \tag{17}$$

$$c_{CO_3^{2-}} = \frac{Q_C \cdot K_{C2}}{c_{H^+} + K_{C2}} \tag{18}$$

Einsetzen von (17) und (18) in (15) und (16) und Auflösen nach $c_{NH_4^+}$ führt zu

$$c_{H^+} = \frac{B + \sqrt{B^2 + 4AC}}{2A} \text{ (nur positives Vorzeichen sinnvoll) } pH = -\log c_{H^+} \tag{19}$$

$$A = \kappa^* - Q_C \cdot c, \ \kappa^* = \frac{\kappa}{\Lambda_{NH4}}, \ c = \frac{\Lambda_{HCO3}}{\Lambda_{NH4}} + 1, \ C = K_{C2} \cdot K_W \cdot b$$

$$B = K_W \cdot b - K_{C2}(\kappa^* - Q_c \cdot d), \ b = \frac{\Lambda_{OH}}{\Lambda_{NH4}} + 1, \ d = \frac{\Lambda_{CO3}}{\Lambda_{NH4}} + 2$$

Einsetzen von $c_{H^+}$ in das Gleichungssystem führt zu $Q_N$:

$$Q_N = \left\{ \frac{K_W}{c_{H^+}} + \frac{Q_C(c_{H^+} + 2K_{C2})}{c_{H^+} + K_{C1}} \right\} \cdot \left\{ \frac{K_N}{c_{H^+}} + 1 \right\} \tag{20}$$

Vorteilhafterweise wird weiterhin die Temperatur des Probestroms gemessen. Zu diesem Zweck können eine oder mehrere Temperatur-Meßvorrichtungen vorgesehen sein. Dies dient dazu, die ermittelten chemischen Parameter auf eine Bezugstemperatur umzurechnen. Das hat den Vorteil, daß der Temperatureinfluß auf die analytischen Größen, welche an chemischen Gleichgewichten teilnehmen berücksichtigt werden kann. Dieser Temperatureinfluß ist prinzipiell durch rein apparative Vorkehrungen nicht korrigierbar. Die sogenannte Temperaturkompensation bei Leitfähigkeitsmeßgeräten setzt nämlich die Kenntnis des Gleichgewichtssystems und des zugehörigen Temperaturkoeffizienten voraus. Der Temperatureinfluß auf Meßgrößen läßt sich jedoch mit Hilfe eines bei der vorliegenden Erfindung verwendeten Prozessors korrekt rechnerisch berücksichtigen, wenn zu den analytischen Größen gleichzeitig die Temperatur des Probestroms gemessen wird, und die Temperaturabhängigkeit der Gleichgewichtskonstanten und der Äquivalentleitfähigkeiten programmiert sind und dem Prozessor zur Verfügung stehen. Die Umrechnung der Meßwerte von Meßtemperatur auf Bezugstemperatur ist an sich bekannt und erfolgt mit einfachen Polynomen der Form

$$k = a + b \cdot T + c \cdot T^2 \ldots \tag{21}$$

oder mit der van t'Hoff'schen Gleichung

$$\log \frac{K}{K_0} = \frac{\Delta H}{4{,}57}\left(\frac{T_0 - T}{T_0 \cdot T}\right). \tag{22}$$

In einer weiteren Ausbildung der Erfindung kann ein Messen des pH-Wertes des Probestroms mittels einer pH-Wert-Meßvorrichtung vorgesehen sein. Hierdurch wird also der pH-Wert unabhängig von dem rechnerischen Ermitteln des pH-Wertes bestimmt. Diese pH-Wert-Meßvorrichtung kann ebenfalls mikroprozessor-gesteuert sein. Diese pH-Wert-Messung kann alternativ zu oder zusammen mit dem rechnerischen Ermitteln des pH-Wertes erfolgen.

Das oben beschriebene rechnerische Ermitteln des pH-Wertes und die oben beschriebene Messung des pH-Wertes sind nicht nur für sich allein von Bedeutung. Es ist nämlich auch möglich, diese beiden auf unterschiedlicher Weise erhaltenen pH-Werte miteinander zu vergleichen, so daß ein "pH-Wert-Vergleichsparameter" ermittelt wird, welcher Rückschlüsse auf betriebsmäßige oder meßtechnische Störungen erlaubt. Dieser Vergleich kann dann automatisch durch den Mikroprozessor durchgeführt werden. Wird bei diesem Vergleich zwischen errechnetem und gemessenen pH-Wert eine Differenz festgestellt, dann wird dies entweder durch eine meßtechnische Störung oder durch die Anwesenheit von Fremdionen, z.B. durch eine Kühlwasserleckage, hervorgerufen.

Weiterhin kann auch eine Messung der Konzentration von Natriumionen in dem Probestrom vorgesehen sein. Diese Messung kann kontinuierlich erfolgen. Durch diese Messung läßt sich die Gegenwart von Fremdionen in dem System Ammoniak-Kohlensäure-Wasser berücksichtigen. Bei z.B. bekannter Kühlwasseranalyse dient nämlich Natrium als Pilotion für kationische und anionische Fremdionen. Es gilt dabei:

$$\text{Summe aller Kationen } \Sigma K^+ = f_+ \cdot {}^c Na^+ \tag{23}$$

und

$$\text{Summe aller Anionen } \Sigma A^- = f_- \cdot {}^c Na^+, \tag{24}$$

wobei

${}^c Na^+$ die Natriumionen-Konzentration,

$f_+$ der Proportionalitätsfaktor zwischen der Natriumionen-Konzentration und der Gesamtkonzentration von allen Kationen, und

$f_-$ der Proportionalitätsfaktor zwischen der Natriumionen-Konzentration und der Gesamtkonzentration von allen Anionen ist, wobei

$f_+ > f_-$, da von den Anionen der Anteil an Hydrogencarbonat abzuziehen ist.

Für die Äquivalentleitfähigkeiten können Durchschnittswerte eingesetzt werden.

Für Messungen der Konzentration der Natriumionen kann eine geräteinterne Kalibrierung und Konditionierung des Sensors vorgesehen sein.

Bei Wasserdampfkreisläufen in Kraftwerken kann eine Kondensatreinigungsanlage vorgesehen sein. Diese ist dann im Bypass zum Wasserdampfkreislauf geschaltet und dient bei Blockanfahrten, Kühlwasserleckagen und Überschüssen an Konditionierungsmitteln (z.B. Ammoniak) der Entsalzung bzw. Reinigung des Kondensats und damit des gesamten Wasserdampfkreislaufes. Ist die Kondensatreinigungsanlage zugeschaltet, so wird Ammoniak (in der Form von Ammonium-Ionen) durch ein großes Kationenfilter (oder sog. Ammoniak-Filter) aus dem Kreislauf eliminiert. Der Durchsatz von Kondensat durch die Kondensatreinigungsanlage wird durch Betriebsmessungen kontinuierlich erfaßt. Das zur Aufnahme von Ammoniak vorgesehene Ammoniak-Filter des Entsalzungsteils der Kondensatreinigungsanlage muß in der Regel etwa 14-tägig bis monatlich regeneriert werden. Bei bekannten Anlagen gibt sich der Erschöpfungsgrad durch einen Durchbruch, welcher durch einen plötzlichen Anstieg der Leitfähigkeit zu erkennen ist. Es ist jedoch wünschenswert, die aktuelle Kapazitätsreserve des Ammoniak-Filters zu kennen, um eine fällige Regeneration besser erkennen zu können. In einer vorteilhaften Weiterbildung der Erfindung ist es deswegen vorgesehen, den jeweils aktuellen Erschöpfungsgrad des Ammoniak-Filters der Kondensatreinigungsanlage rechnerisch zu ermitteln. Dies kann aus der Kenntnis der Ammoniak-Konzentration, des Kondensatdurchsatzes und der Betriebszeit der Kondensatreinigungsanlage erfolgen, wobei die hier interessierende Ammoniak-Konzentration vor Eintritt in die Anlage kontinuierlich berechnet wird. Die Berechnung der Kapazitätsreserve ist also möglich mit Hilfe des Prozessors bei Verwendung der ermittelten Ammoniak-Konzentration im Kondensat, des Kondensatdurchsatzes und der Betriebszeit seit der letzten Regeneration. Dabei gilt:

$$C_x = \Sigma Q_N \cdot q_K \cdot \Delta t \tag{25}$$

und

$$\eta = \frac{C_0 - C_x}{C_0}, \tag{26}$$

wobei $C_x$ die verbrachte Kapazität, $Q_N$ die $NH_3$-Konzentration während einer Betriebsperiode, $q_K$ der mittlere Kondensatdurchsatz, $\Delta t$ die Betriebsperiode und $\eta$ die Restkapazität ist.

Nach jeder Regeneration wird die Kapazität $\eta$ auf Eins zurückgesetzt.

Ein weiteres Problem bei Kraftwerken ist die Kühlwasserleckage. Das erfindungsgemäße Verfahren kann deswegen das rechnerische Ermitteln des Ausmaßes einer eventuellen Kühlwasserleckage beinhalten. Dieser Aspekt der Erfindung beruht auf der Erkenntnis, daß es mit Hilfe des Prozessors möglich ist, eine Kühlwasserleckage bei zwei verschiedenen Betriebszuständen zu berechnen. Bei einem ersten Betriebszustand ist die Kondensatreinigungsanlage in Betrieb. Dann gilt

$$q_L = \left(\frac{\lambda_K - \lambda_0}{\lambda_{L\infty}}\right) \cdot q_K, \tag{27}$$

wobei $q_L$ der Leckagefluß (Volumen/Zeit), $q_K$ der Kondensatdurchsatz, $\lambda_K$ die Leitfähigkeit im Kondensat, $\lambda_0$ die Grundleitfähigkeit ohne Kontamination, $\lambda_{L\infty}$ die auf unendliche Verdünnung gerechnete Leitfähigkeit des Kühlwassers (fiktiv gemessen hinter stark saurem Kationenaustauscher) ist.

Bei einem zweiten Betriebszustand ist die Kondensatreinigungsanlage außer Betrieb. Dann gilt für die Konzentrationsänderung im Kondensat

$$\frac{dC}{dt} = \frac{1}{V}(C_L \cdot q_L + C_Z \cdot q_Z) - \frac{1}{V}[q_V + \beta(q_Z - q_V) + (1-\beta)q_S] \cdot C \tag{28}$$

$$Q(t) = C_L \cdot q_L + C_Z \cdot q_Z$$

$$P(t) = q_V + \beta(q_Z - q_V) + (1-\beta)q_S$$

und nach Integration in den Grenzen $C = 0 / t = 0$ für das Ausmaß der Leckage

$$q_L = \frac{PV(\lambda - \lambda_{(0)} \cdot e^{-Pt}) - \lambda_Z \cdot q_Z(1 - e^{-Pt})}{(1 - e^{-Pt})\lambda_{L\infty}} \tag{29}$$

Es gilt

$$q_L + q_Z \approx q_Z; \quad q_Z \approx q_V + q_D; \quad q_K = f_q \cdot q_S \tag{30}$$

und bei

$$\frac{dC}{dt} = 0 \tag{31}$$

für die maximale Leitfähigkeit

$$\lambda_{max} = \frac{\lambda_{L\infty} \cdot q_L + \lambda_Z q_Z}{q_V + \beta(q_Z - q_V) + (1 - \beta)q_S} \tag{32}$$

nach der Zeit

$$t = \frac{1}{P}\ln[PVC - Q)/(PVC_0 - Q)] \tag{33}$$

Hierbei ist V das Systemvolumen, $f_q$ das lastabhängige Verhältnis Speisewasser/Kondensatmenge, $\beta$ der Proportionalanteil der Dampfverluste an der Salinität, $\lambda_{(0)}$ die Kondensatleitfähigkeit bei t = 0 und für die Indizes gilt Z = Zusatzwasser, L = Leckage, K = Kondensat, V = Wasserverluste, D = Dampf und S = Speisewasser.

Damit läßt sich also das Ausmaß einer eventuellen Kühlwasserleckage für die beiden Betriebsfälle "Kondensatreinigung in Betrieb" und "Kondensatreinigung außer Betrieb" aus den gemessenen Daten der Leitfähigkeiten und der Konzentration der Natriumionen berechnen.

Da der pH-Wert eine vorgeschriebene Überwachungsgröße (VGB-Richtwert) ist und als Kriterium für eine geregelte Ammoniak-Dosierung dienen kann, ist eine exakte Kalibrierung einer vorhandenen pH-Wert-Meßvorrichtung von besonderer Bedeutung. Die Kalibrierung von pH-Elektroden muß jedoch relativ häufig vorgenommen werden und erfordert nach der konventionellen Methode unter Benutzung zweier Pufferlösungen einen erheblichen Arbeitsaufwand. In einer Weiterbildung der vorliegenden Erfindung ist es daher vorgesehen, die Kalibrierung der pH-Wert-Meßvorrichtung zu automatisieren. Dies kann durch die in Anspruch 13 aufgeführten Verfahrensschritte erfolgen. Dieses Verfahren der automatischen pH-Kalibrierung macht sich die Tatsache zunutze, daß es in reinen Ammoniak/Ammonium-Lösungen eine eindeutige, temperaturabhängige Korrelation zwischen dem pH-Wert und der Leitfähigkeit gibt. Sind Leitfähigkeit und Temperatur bekannt, läßt sich mit hoher Genauigkeit der pH-Wert berechnen. Die automatische Kalibrierung wird bei neutralem ph-Wert und bei einem beliebigen Punkt im alkalischen Bereich durchgeführt. Damit kann die E/pH-Gerade zu jeder Zeit bestimmt werden. Weiterhin ist es dann vorteilhaft, wenn die Anwesenheit von Kohlensäure meßtechnisch nach der erfindungsgemäßen Methode oder apparativ berücksichtigt wird. Während der Kalibrierung wird der letzte plausible Meßwert gespeichert, damit die Datenerfassung nicht unterbrochen wird und da sonst kein echter Meßwert während einer Kalibrierung vorliegt.

Es kann also durch ein rechnerisches Zurücksetzen des Asymmetriepotentials auf seinen ursprünglichen Wert eine externe Einpunkt-Kalibrierung im alkalischen Bereich mit Pufferlösung ersetzt werden. Wenn ebenfalls dann deionisiertes Probegut von neutralem pH-Wert über eine der Leitfähigkeits-Meßvorrichtungen geführt wird, kann eine externe Zweipunkt-Kalibrierung ersetzt werden.

Durch den zentralen Mikroprozessor kann bei einer Kalibrierung die Korrektur des Nullpunktes und/oder der Steigung der pH-Kurve ebenfalls durch Ansteuerung der entsprechenden Stellglieder der Meßvorrichtung vorgenommen werden.

Es sei noch darauf hingewiesen, daß Fremdionen in hoher Konzentration durch ihren Beitrag zur Leitfähigkeit das Verfahren der Kalibrierung der pH-Wert-Meßvorrichtung stören, da dann der eindeutige Zusammenhang zwischen Leitfähigkeit und pH-Wert von Ammoniak bzw. Deionat nicht mehr gegeben ist. Beim Vorliegen einer solchen Störung durch Kühlwasserleckage, die sich beispielsweise durch Messung der Natriumionen-Konzentration feststellen läßt, muß konventionell kalibriert werden.

Bei der vorliegenden Erfindung kann es wünschenswert sein, die Funktionstüchtigkeit der verwendeten Leitfähigkeits-Meßvorrichtungen zu prüfen. Dies kann durch die in Anspruch 15 aufgeführten Verfahrensschritte erfolgen. Sollten die dabei erhaltenen, von einander unabhängig ermittelten Werte der Leitfähigkeit voneinander abweichen, dann ist die Funktionstüchtigkeit mindestens einer der Leitfähigkeits-Meßvorrichtungen eingeschränkt.

Bei einer potentiometrischen Messung der Natriumionen-Konzentration stellt sich nach einer Konzentrationsänderung der tatsächliche Endwert erst nach einer sog. Ansprechzeit ein, die von dem Ausmaß der Meßwertänderung und dem Zustand der Elektrode abhängt. Die Integration des Natrium-Meßwertes in einem Datenauswertesystem setzt jedoch Gleichzeitigkeit der Signale voraus, was durch längere Ansprechzeiten einer analytischen Größe nicht gegeben ist. Daher ist es in einer Weiterbildung der Erfindung vorgesehen, einen wahrscheinlichen Endwert der Natriumionen-Konzentration innerhalb eines tolerierbaren Echtzeit-Intervalls vorauszurechnen. Dieser Endwert bzw.

wahrscheinliche Echtzeitwert wird aus dem Anstieg der Meßkurve des scheinbaren Meßwertes als Funktion der Meßzeit berechnet. Dabei wird zur Berechnung dieses Endwertes die Tangente im Kurvenbereich bis zu 5 Minuten Meßzeit, vorzugsweise jedoch bis zu 1 Minute Meßzeit verwendet. Empirische Faktoren der zugrundeliegenden Gleichungen sind experimentell aus typischen Ansprechkurven ermittelt worden.

Es ist wünschenswert, daß mit der Elektrode zur Messung der Natriumionen-Konzentration möglichst häufig eine Einpunkt-Kalibrierung durchgeführt wird. Das Asymmetriepotential läßt sich rechnerisch zurücksetzen. Der Einfluß des Zustandes der Elektrode kann eliminiert werden durch häufige Konditionierung mit einer höher konzentrierten Natriumsalz-Lösung, wodurch die Einstellung des Membrangleichgewichts beschleunigt wird. Kalibrierung und Konditionierung einer Elektrode nimmt viel Zeit in Anspruch. Deswegen ist es zweckmäßig, daß zwei Elektroden im Taktbetrieb jeweils messen bzw. kalibriert oder konditioniert werden. Das Vorsehen von zwei Elektroden erlaubt weiterhin eine kurzzeitige simultane Messung der beiden Elektroden während einer Überschneidung der Taktzeiten, wodurch eine Prüfung der Funktionstüchtigkeit der Elektroden bzw. eine Aussage bzgl. der Richtigkeit der Messung möglich ist.

Durch den Mikroprozessor kann dann die Kalibrierung und Konditionierung der Natrium-Meßeinrichtung vollautomatisch durchgeführt werden.

Bei der erfindungsgemäßen Vorrichtung werden vorteilhafterweise alle Meßgeräte in einer kompakten Meßstation angeordnet.

Das erfindungsgemäße Verfahren kann so ausgebildet sein, daß Daten der Meßgeräte von dem Mikroprozessor kontinuierlich erfaßt werden. Durch den Mikroprozessor können die Daten dann statistisch behandelt und ausgegeben bzw. zu analytischen Rechenwerten verarbeitet werden.

Weiterhin können verschiedene Schaltfunktionen, wie z.B. die Betätigung von Armaturen, Ansteuerung von Ventilen und Eingriffe in Meßverstärker von dem Prozessor gesteuert werden.

Durch die Steuerung mittels eines oder mehrerer Mikroprozessoren kann also das erfindungsgemäße Verfahren automatisiert werden, so daß der Betrieb einer erfindungsgemäßen Meßvorrichtung bzw. Meßstation mit einem Minimum an personellem Aufwand möglich ist.

Ausgestaltungen der Erfindung sind Gegenstand der Unteransprüche.

Ein Ausführungsbeispiel der Erfindung ist nachstehend unter Bezugnahme auf die zugehörigen Zeichnungen näher erläutert.

**Kurze Beschreibung der Zeichnungen**

Fig. 1     ist eine schematische Darstellung und zeigt ein Ausführungsbeispiel einer erfindungsgemäßen Meßvorrichtung in einer ersten Schaltstellung.

Fig. 2     ist eine schematische Darstellung und zeigt die Meßvorrichtung von Fig. 1 in einer zweiten Schaltstellung.

Fig. 3     ist eine schematische Darstellung und zeigt die Meßvorrichtung von Fig. 1 in einer dritten Schaltstellung.

Fig. 4     zeigt in einem Diagramm temperaturabhängige Gleichgewichtskonstanten des Systems $NH_3$ - $CO_2$ - $H_2O$.

Fig. 5     zeigt in einem Diagramm die Temperaturabhängigkeit der Äquivalentleitfähigkeiten.

Fig. 6     zeigt in einem Diagramm ein Beispiel von gemessenen und berechneten Parameter während einer Anfahrt in einem Kraftwerk.

Fig. 7     zeigt in einem Diagramm die temperaturabhängige Korrelation zwischen dem pH-Wert und der Leitfähigkeit in reinen Ammoniak/Ammonium-Lösungen.

Fig. 8     ist eine schematische Darstellung und zeigt das Taktschema einer Zwei-Elektroden-Meßvorrichtung.

Fig. 9     zeigt in einem Diagramm die Berechnung eines vorläufigen Endwertes der Konzentration von Natrium aus der Anfangssteigung der Meßkurve.

**Bevorzugte Ausführung der Erfindung**

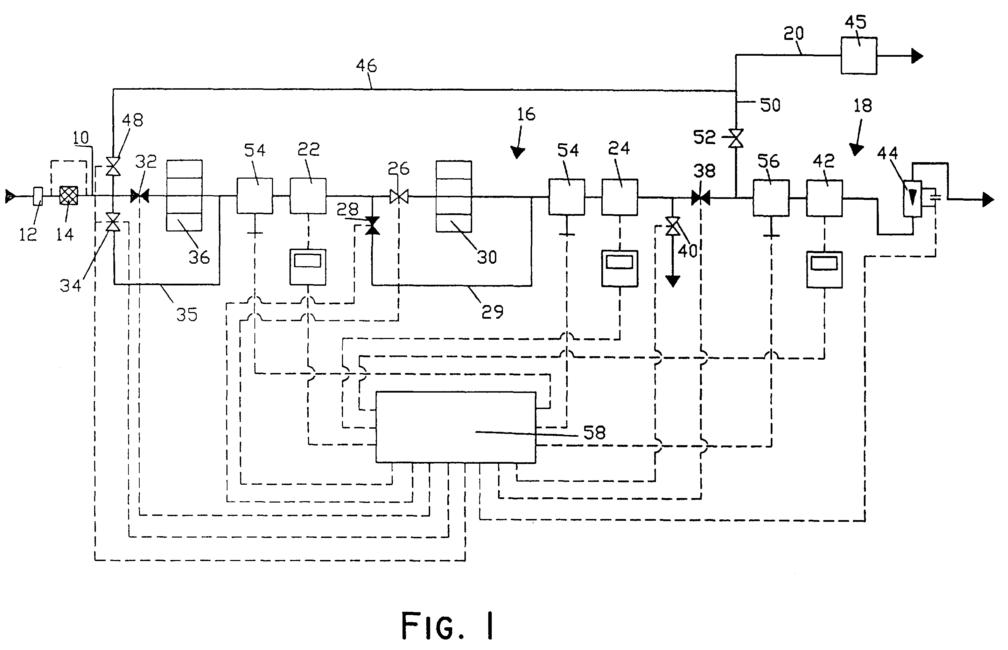

Der prinzipielle Aufbau der Meßvorrichtung ist in Fig. 1 bis 3 gezeigt. Der Probestrom wird von dem zu überwachenden Wasserdampfkreislauf über einen Leitungsstrang 10 entnommen. Diese Leitung 10 enthält ein Feinfilter 12 und einen Durchflußwächter 14. Im Meßbetrieb wird der Probestrom hinter dem Feinfilter 12 und dem Durchflußwächter 14 auf drei Leitungsstränge 16, 18 und 20 verteilt.

In dem ersten Leitungsstrang 16 befinden sich eine erste und eine zweite Leitfähigkeits-Meßzelle 16 bzw. 18. Über ein erstes Ventilsystem mit zwei Ventilen 26 und 28 ist ein stark sauren Kationenaustauscher 30 in den ersten Leitungsstrang 16 einschaltbar. Über eine von dem Ventil 28 gesteuerte Umgehungsleitung 29 (Bypass-Leitung) kann der Probestrom an dem Kationenaustauscher 30 vorbeigeleitet werden. Über ein zweites Ventilsystem mit zwei Ventilen 32 und 34 ist ein stark basischer Anionenaustauscher 36 in den ersten Leitungsstrang 16 einschaltbar. Über eine von dem Ventil 34 gesteuerte Umgehungsleitung 35 (Bypass-Leitung) kann der Probestrom an dem Anionenaustauscher 36 vorbeigeleitet werden. Über ein drittes Ventilsystem mit zwei Ventilen 38 und 40 ist das stromabwärtige Ende des ersten Leitungsstrangs 16 wahlweise mit einem Abfluß (über Ventil 40) oder mit dem (noch zu beschreibenden) zweiten Leitungsstrang 18 (über Ventil 38) verbindbar.

In dem zweiten Leitungsstrang 18 befinden sich eine pH-Wert-Meßvorrichtung 42 und ein Strömungskonstanthalter 44. In dem dritten Leitungsstrang 20 befindet sich eine Natriumionen-Konzentration-Meßvorrichtung 45. Über eine Leitung 46 mit einem Ventil 48 ist die Natriumionen-Konzentration-Meßvorrichtung 45 mit dem Leitungsstrang 10 verbindbar. Über die Leitung 46 und eine Leitung 50 mit einem Ventil 52 ist der dritte Leitungsstrang 18 mit dem Leitungsstrang 10 verbindbar.

Eine erste Temperatur-Meßvorrichtung 54 befindet sich in dem ersten Leitungsstrang 16. Eine zweite Temperatur-Meßvorrichtung 56 befindet sich in dem zweiten Leitungsstrang 18.

Die Meßvorrichtung enthält weiterhin einen zentralen Prozessor 58. Der Prozessor 58 steuert die Ventile 26, 28, 32, 34, 38, 40, 48 und 52, den Strömungskonstanthalter 44 und die Meßinstrumente (die Leitfähigkeits-Meßzellen 22 und 24, die pH-Wert-Meßvorrichtung 42, die Natriumionen-Konzentration-Meßvorrichtung 45 und die Temperatur-Meßvorrichtungen 54 und 56). Weiterhin erhält der Prozessor 58 Meßdaten von den Meßinstrumenten und von dem Strömungskonstanthalter 44. Die Steuer- und Datenleitungen des Prozessors 58 sind in Fig. 1 bis 3 schematisch durch gestrichelte Linien dargestellt.

Die einzelnen Komponenten der Meßvorrichtung sowie die Steuerung und Regelung mittels eines Prozessors sind an sich bekannt und werden daher hier nicht näher beschreiben.

In der in Fig. 1 dargestellten ersten Schaltstellung der Meßvorrichtung (Funktion "Messung") fließt der Probestrom durch alle drei Leitungsstränge 16, 18 und 20. Die Ventile 48 und 52 sind in Offenstellung, so daß die Leitungsstränge 18 und 20 mit dem Leitungsstrang 10 verbunden sind. In dem Leitungsstrang 16 ist sind die Ventile 28, 32 und 38 in Schließstellung und die Ventile 26, 34 und 40 in Offenstellung. Dadurch wird der Anionenaustauscher 36 über die Umgehungsleitung 35 überbrückt. Bei dieser ersten Schaltstellung fließt also der Probestrom in dem ersten Leitungsstrang 16 durch die erste Leitfähigkeits-Meßzelle 22, durch den Kationenaustauscher 30, durch die zweite Leitfähigkeits-Meßzelle 24 und dann ab in den Abfluß über das Ventil 40.

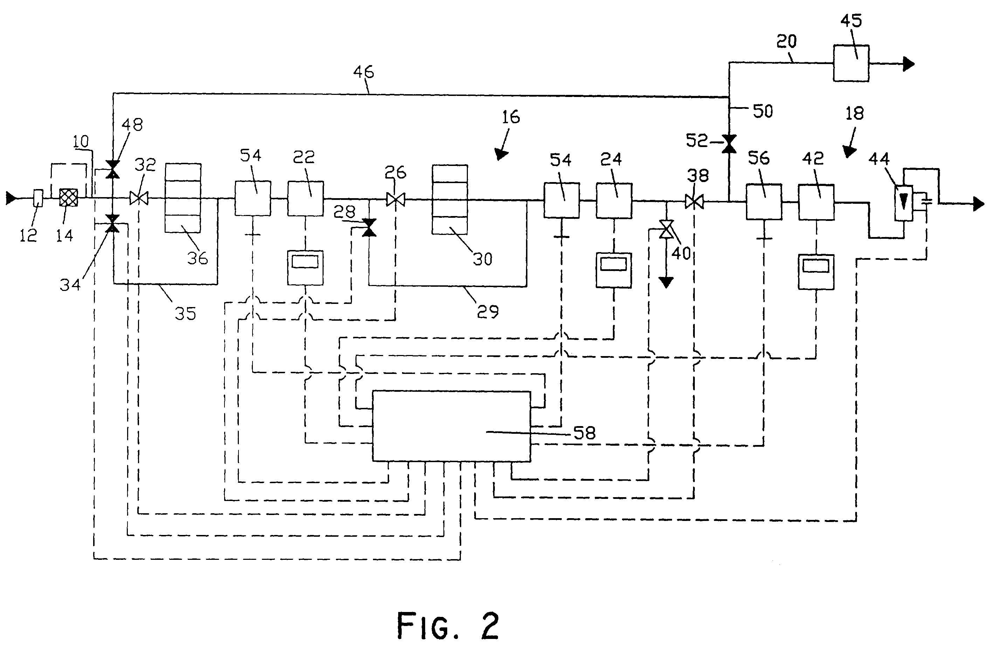

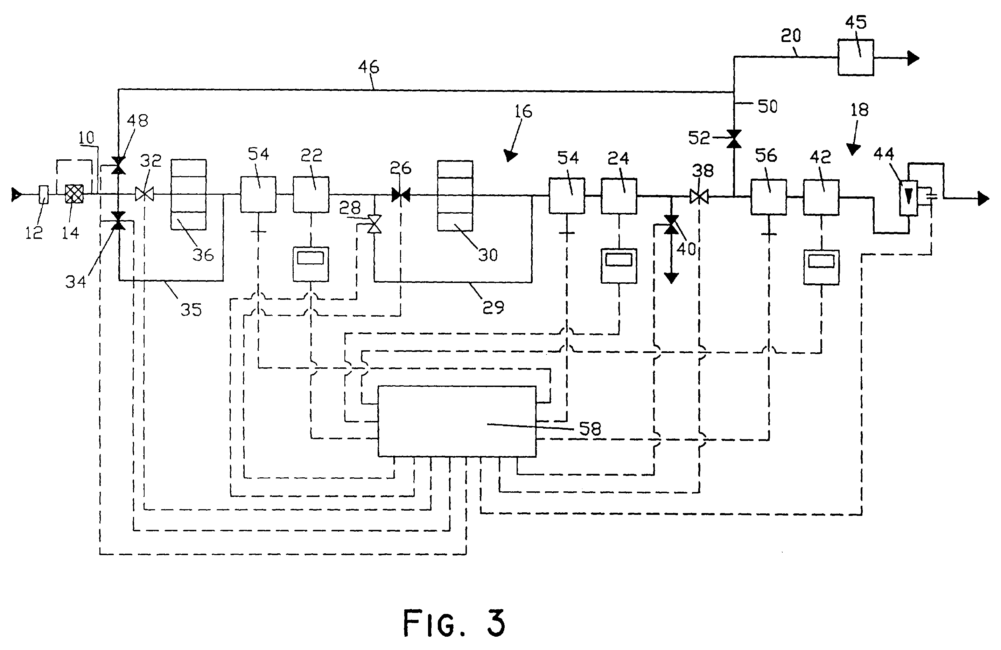

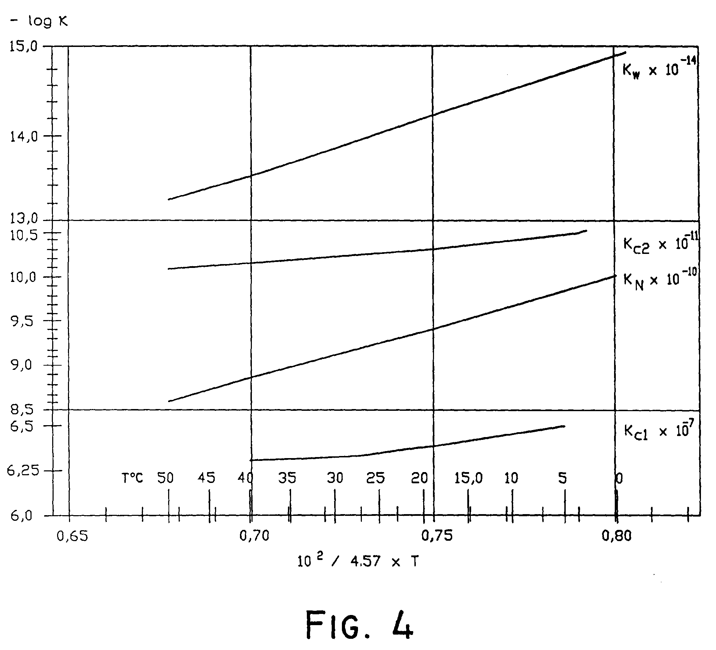

In der in Fig. 2 dargestellten zweiten Schaltstellung der Meßvorrichtung (Funktion "pH-Kalibrierung bei Neutralpunkt") und in der in Fig. 3 dargestellten dritten Schaltstellung der Meßvorrichtung (Funktion "pH-Kalibrierung im alkalischen Bereich") sind die Ventile 48 und 52 in Schließstellung. Dadurch ist der dritte Leitungsstrang 20 von dem Leitungsstrang 10 getrennt. Es findet also keine Messung der Natriumionen-Konzentration statt. Das Ventil 38 befindet sich in Offenstellung. Dadurch ist der Leitungsstrang 18 nicht direkt mit dem Leitungsstrang 10 verbunden, sondern über den ersten Leitungsstrang 16. Das Ventil 32 ist in Offenstellung und das Ventil 34 in Schließstellung, d.h. der Anionenaustauscher 36 ist in den ersten Leitungsstrang 16 eingeschaltet. Weiterhin ist das Ventil 40 in Schließstellung, so daß der Probestrom in dem ersten Leitungsstrang 16 nicht über das Ventil 40 in den Abfluß abfließt. Bei diesen beiden Schaltstellungen (Fig. 2 und 3) der Meßvorrichtung fließt also der Probestrom zunächst durch den ersten Leitungsstrang 16 und dann durch den zweiten Leitungsstrang 18.

In der zweiten Schaltstellung (Fig. 2) der Meßvorrichtung ist das Ventil 26 in Offenstellung und das Ventil 28 in Schließstellung, d.h. der Kationenaustauscher 30 ist in den ersten Leitungsstrang 16 eingeschaltet. In der dritten Schaltstellung (Fig. 3) der Meßvorrichtung ist das Ventil 26 in Schließstellung und das Ventil 28 in Offenstellung, d.h. der Kationenaustauscher 30 wird über die Umgehungsleitung 29 überbrückt und wird also nicht von dem Probestrom durchflossen.

Wenn die Meßvorrichtung sich in der ersten Schaltstellung (Fig. 1) befindet, finden Messungen der elektrischen Leitfähigkeit des Probestroms sowohl stromaufwärts (durch die Leitfähigkeits-Meßzelle 22) als auch stromabwärts (durch die Leitfähigkeits-Meßzelle 24) von dem Kationenaustauscher 30 statt. Weiterhin findet sowohl eine Messung des pH-Wertes des Probestroms durch die pH-Wert-Meßvorrichtung 42 als auch eine Messung der Natriumionen-Konzentration des Probestroms durch die Natriumionen-Konzentration-Meßvorrichtung 45 statt, wobei die Fremdionen-Konzentration in dem Probestrom aus der Messung der Natriumionen-Konzentration durch den Prozessor 58 rechnerisch ermittelt wird. Diese Schaltstellung beinhaltet die normale Meßfunktion der Meßvorrichtung.

Aus den Messungen der elektrischen Leitfähigkeit in dieser ersten Schaltstellung werden die Konzentrationen von Ammoniak und Kohlensäure durch den Prozessor 58 wie oben dargestellt (s. Gleichungen (9) - (20) bzw. (1) - (8)) rechnerisch ermittelt. Weiterhin wird aus diesen Messungen der elektrischen Leitfähigkeit der pH-Wert des Probestroms durch den Prozessor 58 wie oben dargestellt (s. insbesondere Gleichung (19)) rechnerisch ermittelt.

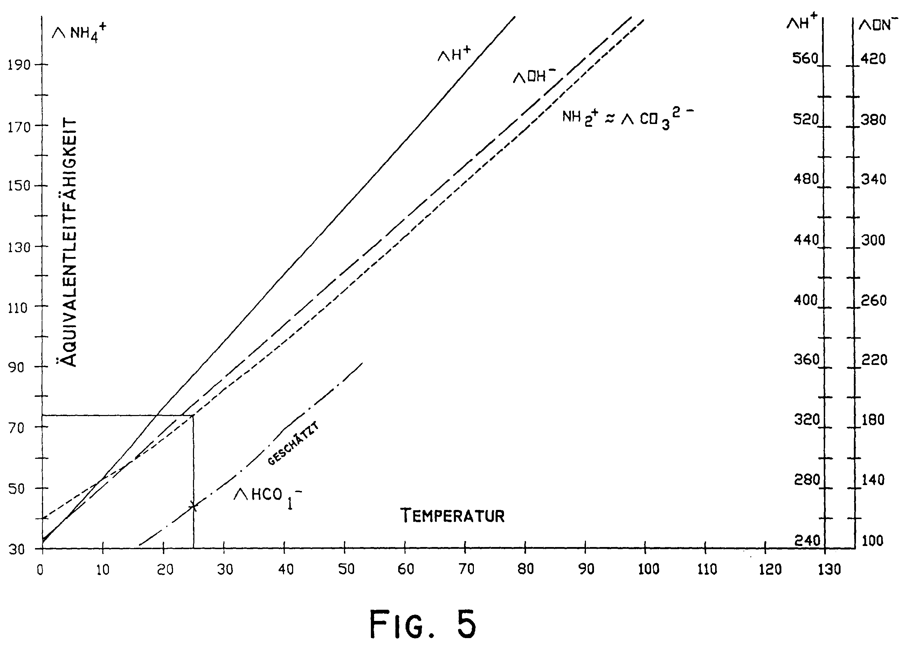

In der ersten Schaltstellung (Fig. 1) der Meßvorrichtung wird die Temperatur des Probestroms einmal durch die Temperatur-Meßvorrichtung 54 und zum anderen durch die Temperatur-Meßvorrichtung 56 gemessen. Die gemessenen Temperaturen werden wie oben dargestellt (vgl. Gleichungen (21) und (22)) zur Umrechnung der gemessenen Größen auf eine Bezugstemperatur herangezogen. Solche Umrechnungen sind an sich bekannt und werden hier nicht näher beschrieben. Fig. 4 und 5 zeigen die bei diesem Verfahren programmierte Temperaturabhängigkeit der Gleichgewichtskonstanten bzw. Temperaturabhängigkeit der Äquivalentleitfähigkeiten.

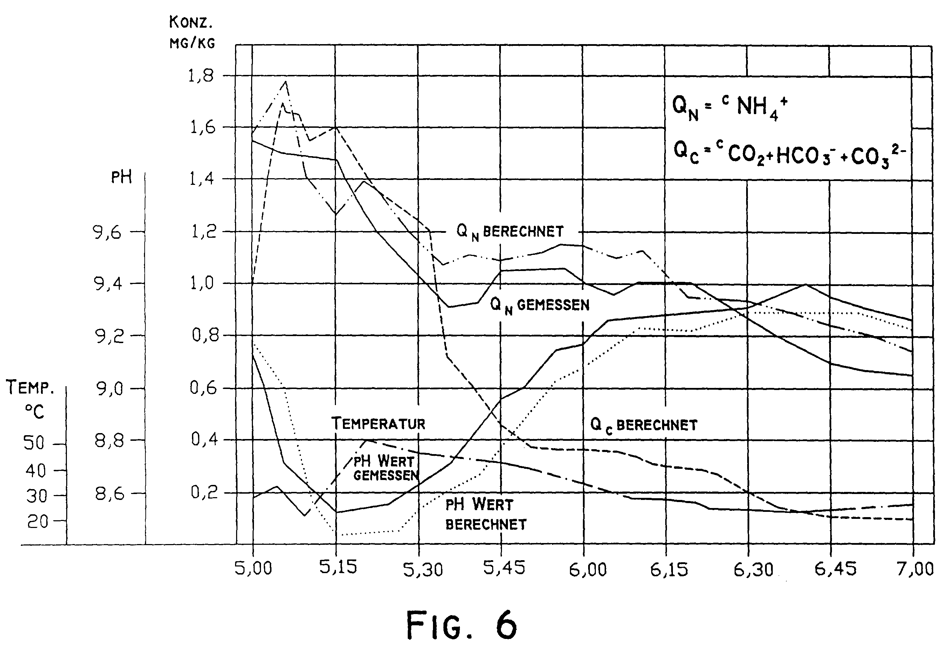

Die Messung des pH-Wertes durch die pH-Wert-Meßvorrichtung 42 sowie die Messung der Natriumionen-Konzentration durch die Natriumionen-Konzentration-Meßvorrichtung 45 sind ebenfalls an sich bekannt und werden hier nicht im Einzelnen beschreiben. In Fig. 6 sind verschiedene gemessene und berechnete analytische Parameter während einer Anfahrt dargestellt.

Durch den Prozessor 58 wird weiterhin der durch die pH-Wert-Meßvorrichtung 42 gemessene pH-Wert mit dem aus den Leitfähigkeitsmessungen berechneten pH-Wert verglichen. Aus diesem Vergleich werden dann durch entsprechende Programmierung des Prozessors 58 Rückschlüsse auf betriebsmäßige oder meßtechnische Störungen gezogen.

Der Prozessor 58 dient ebenfalls zur Bestimmung des Kondensatdurchsatzes und der Betriebszeit einer (in den Figuren nicht gezeigten) Kondensatreinigungsanlage. Aus diese Bestimmung wird dann zusammen mit der Messung der Ammoniak-Konzentration der jeweils aktuelle Erschöpfungsgrad des Ammoniak-Filters der Kondensatreinigungsanlage durch den Prozessor rechnerisch ermittelt.

Der Prozessor 58 dient weiterhin zur Ermittlung des Ausmaßes einer eventuellen Kühlwasserleckage, wie oben schon beschreiben.

Bei Bedarf wird die Meßvorrichtung in die zweite bzw. dritte Schaltstellung umgeschaltet. Dieses Umschalten kann automatisch durch den Prozessor 58 durch Ansteuerung der entsprechenden Ventile erfolgen. Die zweite und dritte Schaltstellung der Meßvorrichtung dienen zur Kalibrierung der pH-Wert-Meßvorrichtung 42.

In der zweiten Schaltstellung (Fig. 2) der Meßvorrichtung findet eine pH-Kalibrierung bei Neutralpunkt, d.h. bei pH=7 statt. Der Probestrom durchläuft den Anionenaustauscher 36 und den Kationenaustauscher 30 bevor er zu der pH-Wert-Meßvorrichtung 42 gelangt. Dadurch ist der Probestrom deionisiert und muß nach rechnerischer Korrektur auf die Bezugstemperatur einen pH-Wert gleich 7 und eine Leitfähigkeit gleich 0,04 $\mu$S/cm haben. Bei einer Abweichung wird das Potential der pH-Elektrode im Neutralpunkt durch den Prozessor 58 rechnerisch oder alternativ durch Eingriff in den Meßverstärker korrigiert.

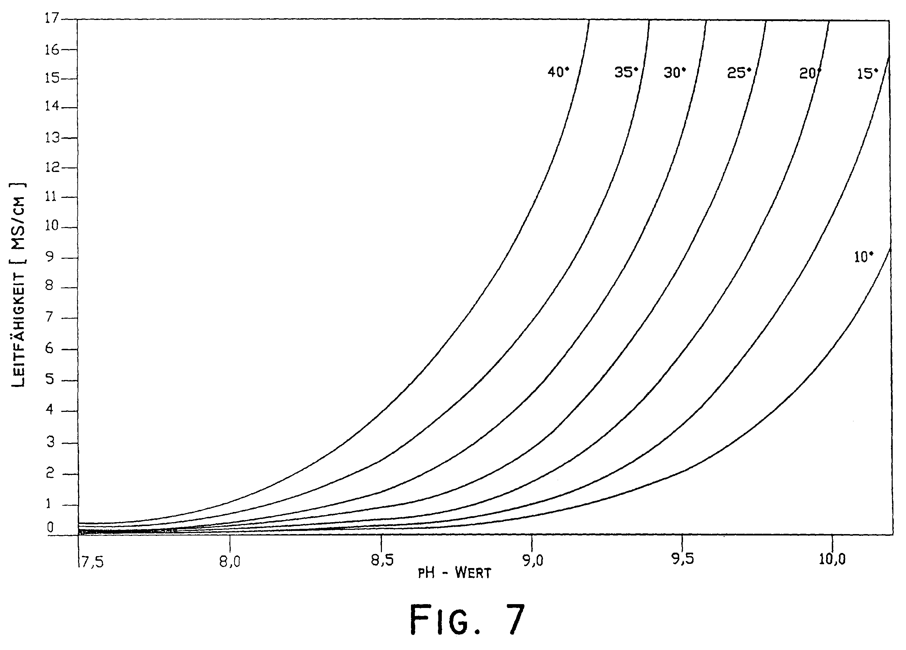

In der dritten Schaltstellung (Fig. 2) der Meßvorrichtung findet eine pH-Kalibrierung im alkalischen Bereich, d.h. bei pH>9 statt. Der Probestrom durchläuft den Anionenaustauscher 36, wird aber durch die Umgehungsleitung 29 an den Kationenaustauscher 30 vorbeigeleitet, bevor er zu der pH-Wert-Meßvorrichtung 42 gelangt. Die auf die Bezugstemperatur korrigierten Anzeigen der Leitfähigkeits- und pH-Messungen korrelieren dann mit der Ammoniak/Ammonium-Konzentration des Probestroms. Bei einer Abweichung wird das Potential der pH-Elektrode in dem gewählten Kalibrierpunkt im alkalischen Bereich durch den Prozessor 58 rechnerisch oder alternativ durch Eingriff in den Meßverstärker korrigiert. Die eindeutige temperaturabhängige Korrelation zwischen dem pH-Wert und der Leitfähigkeit in reinen Ammoniak/Ammonium-Lösungen ist in Fig. 7 gezeigt.

In der dritten Schaltstellung (Fig. 3) der Meßvorrichtung werden ebenfalls die beiden Leitfähigkeits-Meßzellen 22 und 24 durch den Prozessor 58 auf Funktionstüchtigkeit geprüft. In dieser Schaltstellung müssen nämlich die Anzeigen der beiden Leitfähigkeits-Meßzellen 22 und 24 identisch sein. Diese Übereinstimmung dient als Prüfkriterium für die Richtigkeit der Messung.

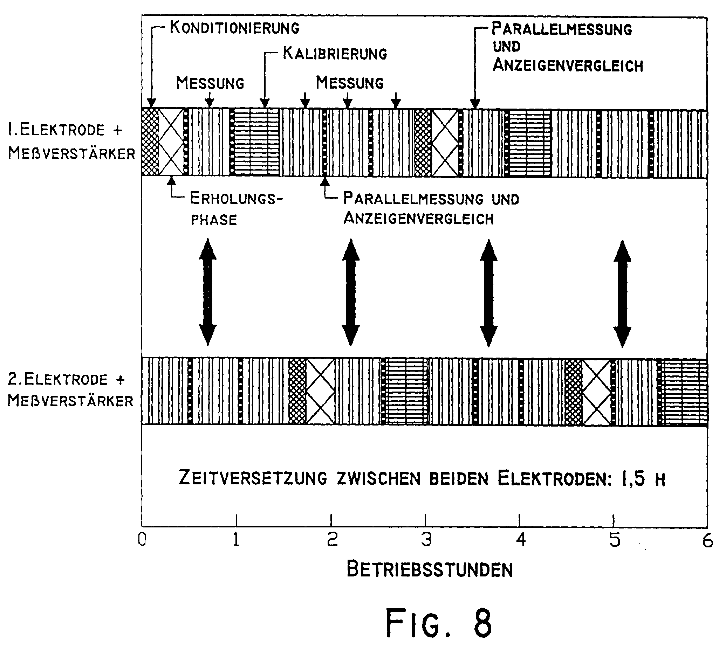

Bei der Natriumionen-Konzentration-Meßvorrichtung 45 handelt es sich um eine übliche Natriumionen-Konzentration-Meßvorrichtung mit zwei Meßsonden und Meßverstärker im Zwillingbetrieb wie oben dargestellt. Dieser Zwillingbetrieb ist in Fig. 8 schematisch dargestellt.

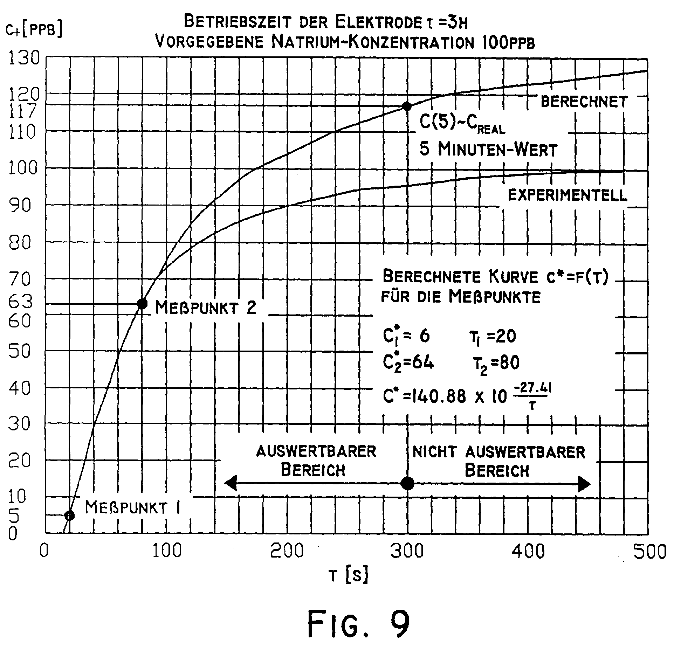

Durch den Prozessor 58 wird ein wahrscheinlicher Echtzeitwert der Natriumionen-Konzentration nach dem oben beschriebenen Verfahren berechnet. Aus dem Anstieg der Ansprechkurve in der Nähe des Ursprungs berechnet der Prozessor 58 nach einer exponentiellen Funktion den wahrscheinlichen Endwert der Messung innerhalb einer tolerierbaren Fehlerbreite. Dies ist in Fig. 9 dargestellt. Dabei gilt:

$$c^* = c(t) \cdot e^{-\frac{t}{z}},$$

wobei c(t) die tatsächliche Konzentrationsänderung, $c^*$ die scheinbare Konzentration (Ablesewert), t die Meßzeit und z die Ansprechkonstante ist.

**Liste verwendeter Symbole**

| | |
|---|---|
| $c^*_i$ [Mol/l]: | Konzentration hinter Kationenaustauscher |
| $c_i$ [Mol/l]: | Konzentration vor Kationenaustauscher |
| $Q_C$ [Mol/l]: | Gesamt-Kohlensäure |
| $Q_N$ [Mol/l]: | Gesamt-Ammoniak |
| $\lambda$ [µS/cm]: | Leitfähigkeit hinter Kationenaustauscher |
| $\kappa$ $\kappa$ [µS/cm]: | Leitfähigkeit vor Kationenaustauscher |
| $K_{C1}$: | 1. Dissoziations-Konstante $CO_2$ |
| $K_{C2}$: | 2. Dissoziations-Konstante $CO_2$ |
| $K_N$: | Dissoziations-Konstante $NH_3$ |
| $K_W$: | Dissoziations-Konstante $H_2O$ |
| $\Lambda$: | Äquivalentleitfähigkeit |
| k: | Temperaturabhängige Gleichgewichtskonstante (Polynom) |
| K: | Temperaturabhängige Gleichgewichtskonstante (van t'Hoff) |
| $K_0$: | Gleichgewichtskonstante unter Referenzbedingungen |
| T: [°C]: | Meßtemperatur |
| $T_0$: [°C]: | Referenztemperatur |
| $f_+$: | Proportionalitätsfaktor Natrium-/Gesamtkonzentration |
| $f_-$: | Proportionalitätsfaktor Natrium-/Gesamtkonzentration |

(Weitere Symbole sind im Text definiert)

**Patentansprüche**

1. Verfahren zur analytischen Bestimmung von chemischen Parameter in ammoniakalisierten Wasser- bzw. Wasserdampf-Kreisläufen, bei welchem ein Probestrom dem Kreislauf entnommen wird, mit den Verfahrensschritten:

   (a) Messen der elektrischen Leitfähigkeit des Probestroms stromaufwärts von einem Kationenaustauscher (30),

   (b) Messen der elektrischen Leitfähigkeit des Probestroms stromabwärts von dem Kationenaustauscher (30), **gekennzeichnet durch**

   (c) rechnerisches mikroprozessor-gesteuertes Ermitteln von chemischen Parameter des Probestroms aus den Messungen der elektrischen Leitfähigkeit des Probestroms.

2. Verfahren nach Anspruch 1, **dadurch gekennzeichnet, daß** aus den Messungen der elektrischen Leitfähigkeit die Konzentration von Ammoniak in ional und gasförmig gelöster Form in dem Probestrom rechnerisch ermittelt wird.

3. Verfahren nach Anspruch 1 oder 2, **dadurch gekennzeichnet, daß** aus den Messungen der elektrischen Leitfähigkeit die Konzentration von Kohlensäure in ional und gasförmig gelöster Form in dem Probestrom rechnerisch ermittelt wird.

4. Verfahren nach einem der Ansprüche 1-3, **dadurch gekennzeichnet, daß** aus den Messungen der elektrischen Leitfähigkeit der pH-Wert des Probestroms rechnerisch ermittelt wird.

5. Verfahren nach einem der Ansprüche 1-4, **gekennzeichnet durch**

   (a) Messen der Temperatur des Probestroms, und

   (b) Umrechnen der ermittelten chemischen Parameter auf eine Bezugstemperatur.

6. Verfahren nach einem der Ansprüche 1-5, **gekennzeichnet durch** Messen des pH-Wertes des Probestroms mittels einer pH-Wert-Meßvorrichtung (42).

**7.** Verfahren nach Anspruch 6, **dadurch gekennzeichnet, daß** die pH-Wert-Meßvorrichtung (42) mikorprozessor-gesteuert ist.

**8.** Verfahren nach Anspruch 6 oder 7, **gekennzeichnet durch**

(a) Vergleichen des rechnerisch ermittelten pH-Wertes mit dem gemessenen pH-Wert des Probestroms, und

(b) Ermitteln eines pH-Wert-Vergleichsparameters, welcher Rückschlüsse auf betriebsmäßige oder meßtechnische Störungen erlaubt.

**9.** Verfahren nach einem der Ansprüche 1-8, **gekennzeichnet durch** Messen der Natriumionen-Konzentration in dem Probestrom.

**10.** Verfahren nach Anspruch 9, **gekennzeichnet durch** rechnerisches Ermitteln der Fremdionen-Konzentration aus der Messung der Natriumionen-Konzentration.

**11.** Verfahren nach einem der Ansprüche 1-10, **gekennzeichnet durch**

(a) Messen der Ammoniak-Konzentration, des Kondensatdurchsatzes und der Betriebszeit einer vorhandenen Kondensatreinigungsanlage, und

(b) rechnerisches Ermitteln der jeweils aktuellen Erschöpfungsgrades des Ammoniak-Filters der Kondensatreinigungsanlage.

**12.** Verfahren nach einem der Ansprüche 1-11, **gekennzeichnet durch** rechnerisches Ermitteln des Ausmaßes einer eventuellen Kühlwasserleckage.

**13.** Verfahren nach einem der Ansprüche 6-12, **gekennzeichnet durch** automatisches Kalibrieren der pH-Wert-Meßvorrichtung (42) mit den Verfahrensschritten:

(a) Messen der Temperatur des Probestroms,

(b) Messen der elektrischen Leitfähigkeit des Probestroms stromabwärts von einem System aus Anionenaustauscher (36) und Kationenaustauscher (30),

(c) Leiten des aus dem System aus Anionenaustauscher (36) und Kationenaustauscher (30) austretenden Probestroms durch die zu kalibrierende pH-Wert-Meßvorrichtung (42),

(d) Berechnen des auf Bezugstemperatur korrigierten, zugehörigen pH-Wertes des Probestroms stromabwärts von dem System aus Anionenaustauscher (36) und Kationenaustauscher (30),

(e) prozessor-gesteuertes Korrigieren des Potentials der pH-Wert-Meßvorrichtung (42) in Abhängigkeit von diesem berechneten pH-Wert des Probestroms stromabwärts von dem System aus Anionenaustauscher (36) und Kationenaustauscher (30),

(f) Messen der elektrischen Leitfähigkeit des Probestroms stromabwärts von einem Anionenaustauscher (36),

(g) Leiten des aus dem Anionenaustauscher (36) austretenden Probestroms durch die zu kalibrierende pH-Wert-Meßvorrichtung (42),

(h) Berechnen des auf Bezugstemperatur korrigierten, zugehörigen pH-Wertes des Probestroms stromabwärts von dem Anionenaustauscher (36), und

(i) prozessor-gesteuertes Korrigieren des Potentials der pH-Wert-Meßvorrichtung (42) in Abhängigkeit von diesem berechneten pH-Wert des Probestroms stromabwärts von dem Anionenaustauscher (36).

**14.** Verfahren nach Anspruch 13, **dadurch gekennzeichnet, daß** das Asymmetriepotential rechnerisch wieder auf seinen ursprünglichen Wert zurückgesetzt wird.

**15.** Verfahren nach einem der Ansprüche 1-14, **gekennzeichnet durch**

(a) Vorbeileiten des Probestroms an dem Kationenaustauscher (30),

(b) Messen der elektrischen Leitfähigkeit des Probestroms mittels eines stromaufwärts von dem Kationenaustauscher (30) vorgesehenen Leitfähigkeits-Meßvorrichtung (22),

(c) Messen der elektrischen Leitfähigkeit des Probestroms mittels eines stromabwärts von dem Kationenaustauscher (30) vorgesehenen Leitfähigkeits-Meßvorrichtung (24), und

(d) Vergleichen der beiden so erhaltene Meßwerte der elektrischen Leitfähigkeit des Probestroms.

**16.** Verfahren nach einem der Ansprüche 9-15, **gekennzeichnet durch** Berechnen eines wahrscheinlichen Echtzeitwertes der Natriumionen-Konzentration aus dem Anstieg der Ansprechkurve des scheinbaren Meßwertes als Funktion der Meßzeit.

**17.** Verfahren nach Anspruch 16, **dadurch gekennzeichnet, daß** zur Berechnung des wahrscheinlichen Echtzeitwertes der Natriumionen-Konzentration die Tangente im Kurvenbereich bis zu 5 Minuten Meßzeit, vorzugsweise jedoch bis zu 1 Minute Meßzeit verwendet wird.

**18.** Verfahren nach Anspruch 16 oder 17, **dadurch gekennzeichnet, daß** zwei Elektroden im Taktbetrieb arbeiten, wobei die eine mißt während die jeweils andere kalibriert bzw. mit höher konzentrierter Natriumsalzlösung konditioniert wird.

**19.** Verfahren nach Anspruch 18, **gekennzeichnet durch** eine Überschneidung der Taktzeiten der beiden Elektroden, bei welcher die beiden Elektroden parallel messen.

**20.** Vorrichtung zur analytischen Bestimmung von chemischen Parametern in ammoniakalisierten Wasser- bzw. Wasserdampf-Kreisläufen, bei welchem ein Probestrom dem Kreislauf entnommen wird, enthaltend

(a) einen stark sauren Kationenaustauscher (30),

(b) eine erste Leitfähigkeits-Meßvorrichtung (22) stromaufwärts von dem Kationenaustauscher (30),

(c) eine zweite Leitfähigkeits-Meßvorrichtung (24) stromabwärts von dem Kationenaustauscher (30), **gekennzeichnet durch**

(d) einen Mikroprozessor (58), durch welchen chemische Parameter des Wassers bzw. des Wasserdampfes aus den Messungen der elektrischen Leitfähigkeit des Probestroms rechnerisch ermittelbar sind.

**21.** Vorrichtung nach Anspruch 20, **gekennzeichnet durch** eine oder mehrere Temperatur-Meßvorrichtungen (54;56) zur Messung der Temperatur des Probestroms.

**22.** Vorrichtung nach Anspruch 20 oder 21, **gekennzeichnet durch** eine pH-Wert-Meßvorrichtung (42) zur Messung des pH-Wertes des Probestroms.

**23.** Vorrichtung nach Anspruch 22, **gekennzeichnet durch** Mittel (58) zum Vergleich der mittels der pH-Wert-Meßvorrichtung (42) gemessenen pH-Wert mit einem aus Leitfähigkeitsmessungen mittels der beiden Leitfähigkeits-Meßvorrichtungen (22,24) ermittelten pH-Wert.

**24.** Vorrichtung nach einem der Ansprüche 20-23, **gekennzeichnet durch** eine Natriumionen-Konzentration-Meßvorrichtung (45).

**25.** Vorrichtung nach einem der Ansprüche 20-24, **gekennzeichnet durch** einen stark basischen Anionenaustauscher (36).

**26.** Vorrichtung nach einem der Ansprüche 20-25, **gekennzeichnet durch** eine erste Umgehungsleitung (29), durch welche der Probestrom an dem Kationenaustauscher (30) vorbeileitbar ist.

**27.** Vorrichtung nach Anspruch 25 oder 26, **gekennzeichnet durch** eine zweiten Umgehungsleitung (35), durch welche der Probestrom an dem Anionenaustauscher (36) vorbeileitbar ist.

FIG. I

FIG. 2

EP 1 045 245 A2

FIG. 3

EP 1 045 245 A2

FIG. 4

FIG. 5

FIG. 6

FIG. 7

EP 1 045 245 A2

FIG. 8

BETRIEBSZEIT DER ELEKTRODE τ =3H
VORGEGEBENE NATRIUM-KONZENTRATION 100PPB

FIG. 9